# EUROPEAN PATENT SPECIFICATION

(11) **EP 0 413 151 B1**
(45) Date of publication and mention of the grant of the patent: **08.02.1995**
(21) Application number: 90113642.4
(22) Date of filing: 17.07.1990
(51) Int. Cl.: C07D 285/08

(54) **Processes for preparing 2-methoxyimino-2- (5-amino-1,2,4-thiadiazol-3-yl)acetic acid or its salts**
Verfahren zur Herstellung 2-Methoxyimino-2- (5-amino-1,2,4-thiadiazol-3-yl)essigsäure oder ihrer Salze
Procédés pour la préparation de l'acide méthoxyimino-2- (5-amino-1,2,4-thiadiazol-3-yl)acétique ou de ses sels

(30) Priority: 19.07.1989 JP 188228/89
(43) Date of publication of application: 20.02.1991
(73) Proprietor: FUJISAWA PHARMACEUTICAL CO., LTD., Osaka-shi Osaka 541 (JP)
(72) Inventor: Sakane, Kazuo, Kawanishi-shi, Hyogo 666-01 (JP); Terasawa, Takeshi, 1-5-19-503, Toyosato,, Osaka 533 (JP)
(74) Representative: Türk, Gille, Hrabal, Leifert

(56) References cited:
- EP-A- 0 150 609
- EP-A- 0 248 434
- GB-A- 2 094 794

## Description

The present invention relates to a novel preparing method of 2-methoxyimino-2-(5-amino-1,2,4-thiadiazol-3-y1)acetic acid or its salts. The object of this invention is to provide a novel industrial preparing method excellent in yield, purity, etc., of 2-methoxyimino-2-(5-amino-1,2,4-thiadiazol-3-y1)acetic acid or its salts which are useful as acylating agents for preparing 7-acylaminocephalosporin compounds having an excellent antimicrobial activity.

GB 2,094,794 discloses a method for producing a carboxylic acid compound by directly hydrolyzing the corresponding nitrile compound having a free amino group. During the process described therein a large amount of impurities is produced together with the object compound.

In EP-A-0 248 434 a process for the preparation of thiadiazolylacetamide derivative is disclosed, which comprises hydrolyzing a nitrile compound, wherein a amino group contained therein may be protected. Hydrolyzing of the nitrile compound results in large amounts of impurities.

EP-A-0 150 609 discloses cephalosporin compounds represented by the general formula (I) and the preparation thereof. The compounds are prepared by directly applying hydrolysis to several nitrile compounds to obtain the corresponding carboxylic acids.

The 2-methoxyimino-2-(5-amino-1,2,4-thiadiazol-3-yl) acetic acid, the object compound prepared by the process of this invention, may be represented by the undermentioned formula: 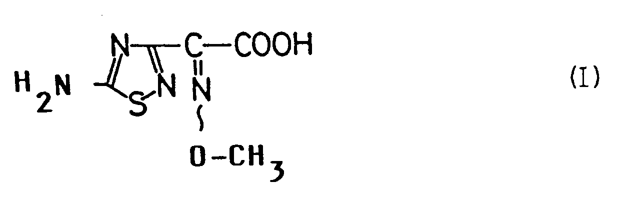
According to this invention, the 2-methoxyimino-2-(5-amino-1,2,4-thiadiazol-3-yl)acetic acid (I)or its salts, the object compounds, are prepared by the following method: 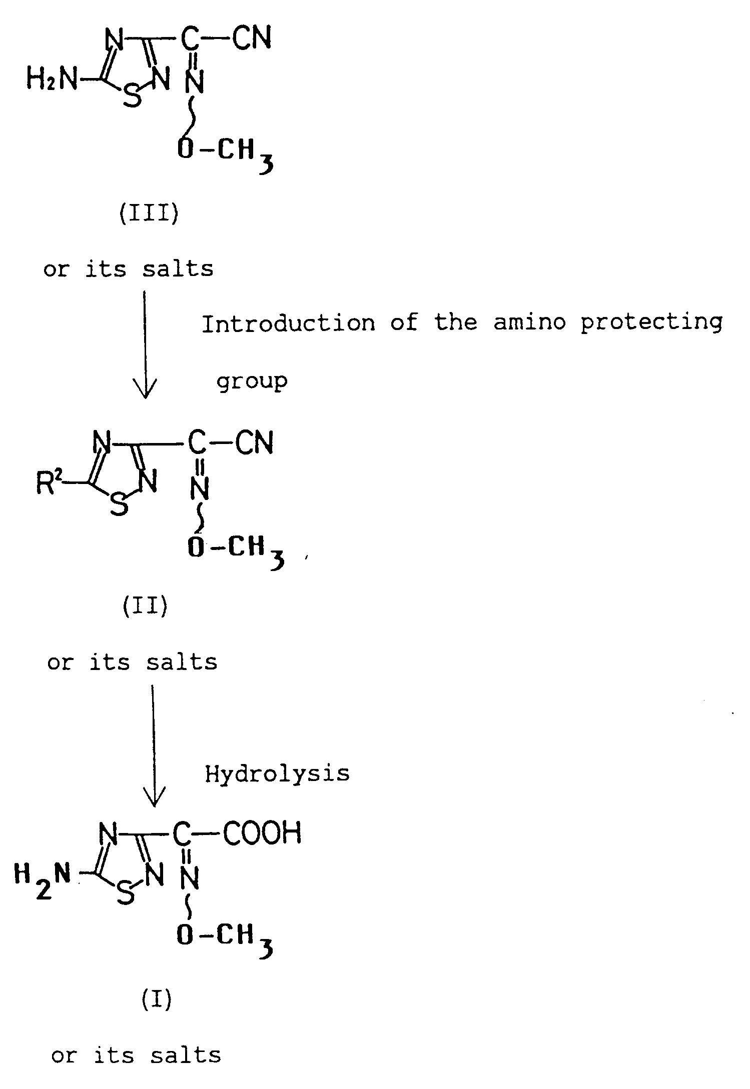
(where R² represents a protected amino group). The starting material (III) is a well-known compound, which may be prepared by a method mentioned in Japanese Laid-Open Patent Publication NO. 57-158769.
With regard to the object compound (I), the intermediate (II) and the starting material (III), the partial structural formula given by 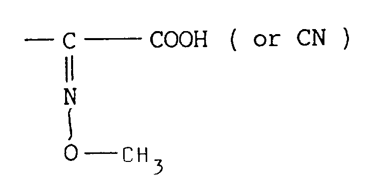
is to be understood to combine both geometric structures shown by the undermentioned formula: 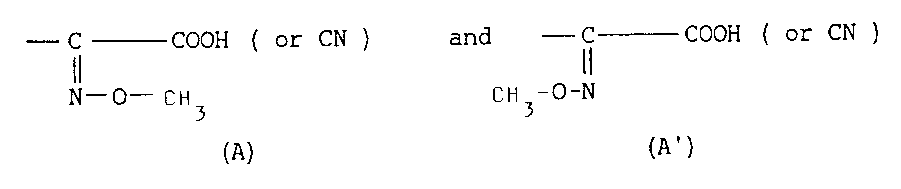
In this Specification, as to all compounds having the above-mentioned partial structures, compounds having the geometric structure given by the formula (A) are designated "syn-isomer", and those having the structure given by (A') "anti-isomer".

Suitable salts of the object compound (I) may include salts with a base and an acid addition salt such as salt with an inorganic base, e.g., an alkali metal salt (e.g., sodium salt, potassium salt, etc.), an alkaline earth metal salt (e.g., calcium salt, magnesium salt, etc.), an ammonium salt, a salt with an organic base, e.g., an organic amine salt (e.g. triethylamine salt, pyridine salt, picoline salt, ethanolamine salt, triethanolamine salt, dicyclohexylamine salt, N,N-dibenzylethylenediamine salt, etc.); an inorganic acid addition salt (e.g., hydrochloride, hydrobromide, sulfate, phosphate, etc.); an organic acid addition salt, e.g., an organic carboxylic or sulfonic acid addition salt (e.g., formate, trifluoroacetate, methanesulfonate, benzenesulfonate, acetate,p-toluenesulfonate,etc.); a salt with a basic amino acid (e.g., arginine, etc.), etc.

Appropriate examples of various definitions included in the scope of this invention, which has been described hereabove and which will be described later in this Specification, will be explained in detail hereunder:

The term "lower" implies groups which have from 1 to 6 carbon atoms, unless otherwise specified.

As the protecting groups in the protected amino groups, the amino protecting groups which are customarily used with cephalosporin compounds, for example, the laterdescribed acyl groups or, for example, benzyl groups, etc., may be mentioned, but acyl groups are preferable.

Suitable acyl groups may include aliphatic acyls, aromatic acyls, heterocyclic acyls and aliphatic acyls substituted by aromatic or heterocyclic groups, etc.

Aliphatic acyl groups may include saturated or unsaturated, noncyclic or cyclic ones and such groups are, e.g., lower alkanoyls, such as formyl, acetyl, propionyl, butyryl, isobutyryl, valeryl, isovaleryl, pivaloyl, hexanoyl, etc., lower alkanesulfonyls such as mesyl, ethanesulfonyl, propanesulfonyl, etc., lower alkoxycarbonyls such as methoxycarbonyl, propoxycarbonyl, butoxycarbonyl, tertiarybutoxycarbonyl, etc., lower alkenoyls such as acryloyl, methacryloyl, crotonoyl, etc., C3-C7 cycloalkanecarbonyls such as cyclohexanecarbonyl, etc., and amidino group, etc.; among them, lower alkanoyl groups are preferable. These acyl groups may contain more than one suitable substituents such as, e.g., halogens including chlorine, bromine and fluorine, cyano, alkyl groups such as methyl, ethyl, propyl, isopropyl, butyl, etc., alkenyl groups such as vinyl, allyl, etc.

The preparing methods of the object compound (I) or its salts are described in detail hereunder:

### Method 1:

The compound (I) or its salts is prepared by hydrolyzing the compound (II) or its salts. This hydrolysis should preferably be performed in the presence of bases or acids. Suitable bases include inorganic and organic bases such as alkali metals (e.g., sodium, potassium, etc.), alkaline earth metals (e.g., magnesium, calcium, etc.), their hydroxides, carbonates or bicarbonates, trialkylamines (e.g., trimethylamine, triethylamine, etc.), picoline, 1, 5-diazabicyclo-[4.3.0] none-5-ene, 1, 4-diazabicyclo-[2.2.2] octane, 1, 8-diazabicyclo-[5.4.0] undecene-7, etc. Suitable acids include organic acids (e.g., formic acid, acetic acid, propionic acid, trifluoroacetic acid, etc.) and inorganic acids (e.g., hydrochloric acid, hydrobromic acid, sulfuric acid, etc.).

The reaction is usually carried out in such a solvent as waters alcohol (e.g., methanol, ethanol, etc.), tetrahydrofuran, dioxane or any of their mixtures, or any of other solvents which do not adversely influence the reaction. Liquid bases or acids may be used as the solvents. The reaction temperature is not critical and the reaction is usually carried out under warming or heating.

Removal of the amino protecting group can be carried out in accordance with a conventional method (e.g. hydrolysis, reduction, etc.), if necessary.

### Method 2:

The compound (II) or its salts can be prepared by reacting amino protecting agents with the respectively corresponding compound (III) or its reactive derivatives at the amino group or its salts.

Amino protecting agents may include acylating agents such as aliphatic, aromatic or heterocycliccarboxylic acids (or sulfonic acids or carbonic acid esters or carbamic acid) and thioacids corresponding thereto and reactive derivatives of these acids and aliphatic, aromatic or heterocyclic isocyanates (or thioisocyanates) etc.; aforementioned reactive derivatives of acids may include, acid halides, acid anhydrides, active amides, active esters, etc.

Preferable reactive derivatives of the compound (III) at the amino group may include Schiff's base type iminos formed by reaction of the compound (III) with such carbonyl compounds as aldehyde, ketone, etc., or their enamine type tautomeric isomers, and silyl derivatives formed by reaction of the compound (III) with silyl compounds such as bis (trimethylsilyl) acetamide, mono (trimethylsilyl) acetamide, e.g.,N-(trimethylsilyl) acetamide, etc., bis (trimethylsilyl) urea, etc.

Preferable examples of the aforementioned reactive derivatives of acids may be acid chlorides; acid asides; mixed acid anhydrides with such acids as substituted phosphoric acids, e.g., dialkyl phosphates, phenyl phosphates, diphenyl phosphates, dibenzyl phosphates, halogenated phosphates, etc., dialkyl phosphites, sulfurous acid, thiosulfuric acid, sulfuric acid, sulfonic acids, e.g., methanesulfonic acid, etc., aliphatic carboxylic acids, e.g., acetic acid, propionic acid, butyric acid, isobutyric acid, pivalic acid, valeric acid, isovaleric acid, 2-ethylbutyric acid, trichloroacetic acid, etc., or aromatic carboxylic acids, e.g., benzoic acid, etc.; symmetric acid anhydrides; active amides with imidazole, 4-substituted imidazoles, dimethylpyrazole, triazole or tetrazole; and active esters, etc.

These reactive derivatives can optionally be selected from among them according to the kind of the acid to be used.

The reaction is carried out in conventional solvents, such as acetone, dioxane, chloroform, methylene chloride, ethylene chloride, tetrahydrofuran, ethyl acetate, N,N-dimethylformamide and pyridine or any other organic solvents which do not adversely influence the reaction. Further, if the acylating agents used are liquid, they also are usable as solvents.

When the acids for use as acylating agents are used in free form or in the form of salts in this reaction, the reaction is preferably carried out in the presence of a conventional condensing agent such as N,N'-dicyclohexylcarbodiimide; N-cyclohexyl-N'-morpholinoethyl carbodiimide: N-cyclohexyl-N'-(4-diethylaminocyclohexyl) carbodiimide; N, N'-dietylcarbodiimide; N,N'-diisopropyl carbodiimide; N-ethyl-N'-(3-dimethylaminopropyl) carbodiimide; or the so-called Vilsmeier reagent prepared by the reaction of N,N-dimethyl formamide with thionyl chloride, phosgene, trichloromethyl chloroformate, phosphorus oxychloride, etc.

The reaction may also be carried out in the presence of inorganic or organic base such as alkali medal hydrogencarbonates, tri-(lower) alkyl amines, pyridine, N-(lower) alkyl morpholine, N, N-di-(lower) alkyl benzyl amines, etc.

The reaction temperature is not critical and the reaction is usually carried out under cooling or warming.

The object of this invention is, as hereabovedescribed, to provide a novel preparing method of the compound (I) or its salts.

This invention is based on the finding that this novel method is industrially superior to the well-known method in such aspects as yield, purity, etc., of the object compound (I) or its salts.

The undermentioned examples are given for the purpose of illustrating this invention in more detail:

### Example 1:

Formic acid (55.24g) was dropped into acetic acid anhydride (81.67g), with stirring continued for 30 min at 30 - 35 °C. To this mixture, was added 2-methoxyimino-2-(5-amino-1,2,4-thiadiazol-3-yl) acetonitrile (syn isomer) (29.31g). The mixture, after stirred for 2.4 hr at 55-60 °C, was cooled down to 5 °C. Into this mixture was dropped diisopropyl ether (160ml), with stirring continued for 15 min at 5 °C. The crystal was separated by filtration and, then, washed with diisopropyl ether (160ml) to give 2-methoxyimino-2-(5-formamido-1,2,4-thiadiazol-3-yl)acetonitrile (syn-isomer) (29.82g).
- IR (Nujol, cm-1):: 3140, 3060,1700,1565
- NMR (DMSO-d6,δ ):: 4.26 (3H,s), 8.88 (1H,s), 13.55 (1H, brs)

### Example 2:

A suspension of 2-methoxyimino-2-(5-formamido-1,2,4-thiadiazol-3-y1)acetonitrile (syn-isomer) (2.11g) in 2N aqueous solution (30ml) of sodium hydroxide was stirred for 1.2 hr at 65 °C. After cooling, the mixture was neutralized with 6N hydrochloric acid, followed by addition of ethyl acetate (30ml). The mixture was adjusted to pH 0.8 with 6N hydrochloric acid and the separated water layer was again extracted with ethyl acetate (10ml). The organic layer put together was washed with a salt water, dried with magnesium sulfate and concentrated under reduced pressure. The formed crystal was separated by filtration and, then, washed with ethyl acetate and diisopropyl ether to give 2-methoxyimino-2-(5-amino-1,2,4-thiadiazol-3-yl) acetic acid (syn-isomer) (772mg).
- IR (Nujol, cm-¹):: 3430, 3260, 3190, 1685, 1615, 1535
- NMR (DMSO-d6,δ ):: 3.95 (3H, s), 8.19 (2H, brs), 13.6 - 14.1 (1H, br)

### Example 3:

Pyridine (5.93g) was added at 5 °C into a suspension of 2-methoxyimino-2-(5-amino-1,2,4-thiadiazol-3-yl)acetonitrile (syn-isomer) (9.15g) in dichloromethane (92ml) and, then, acetyl chloride (5.83g) was dropped into the mixture. The mixture was, then, refluxed for 2.4 hr. After cooling, the dichloromethane was removed by evaporation under reduced pressure. Water was added to the residue and the crystal formed was separated by filtration and, then, washed with water, ethanol and diisopropyl ether to give 2-methoxyimino-2-(5-acetamido-1,2,4-thiadiazol-3-yl) acetonitrile (synisomer) (9.75g).
- IR (Nujol, cm-¹):: 3250, 3210, 1670, 1590, 1560, 1535
- NMR (DMSO-d,δ ):: 2.28 (3H, s), 4.26 (3H,s), 13.38 (1H,br)

### Example 4

A suspension of 2-methoxyimino-2-(5-acetamido-1,2,4-thiadiazol-3-yl)acetonitrile (syn-isomer) (4.51g) in 2N aqueous solution (60ml) of sodium hydroxide was stirred for 5 hr at 65 °C. After cooling, the mixture was adjusted to pH 0.8 with 6N hydrochloric acid and subjected to extraction with ethyl acetate. The extract solution was washed with a salt water (10ml), dried with magnesium sulfate and concentrated under reduced pressure. The crystal formed was separated by filtration, washed with ethyl acetate and diisopropyl ether to give 2-methoxyimino-2-(5-amino-1,2,4-thiadiazol-3-yl)acetic acid (syn-isomer) (2.69g purity 99.14%).
- IR (Nujol, cm-¹):: 3450, 3260, 3130,1720,1615,1535
- NMR (DMSO-d6,δ):: 3.94 (3H,s), 8.19 (2H,brs), 13.5 - 14.2 (1H,br)

## Claims

1. A preparing method of a compound represented by a formula: 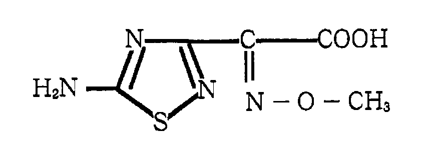 or its salts, which comprises forming a compound represented by a general formula(II): 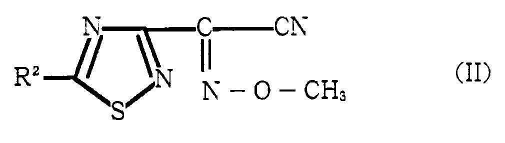 (where R² represents a protected amino group)
or its salts by introducing an amino protecting group into the amino group on the fifth site of a compound represented by a formula: 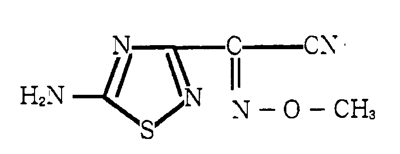 or its salts, and then hydrolyzing a compound(II) or its salts, and if necessary, removing the amino protecting group.

## Patentansprüche

1. Verfahren zur Herstellung einer Verbindung der Formel 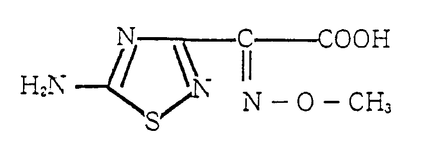 oder ihrer Salze, das umfaßt
die Bildung einer Verbindung der allgemeinen Formel (II) 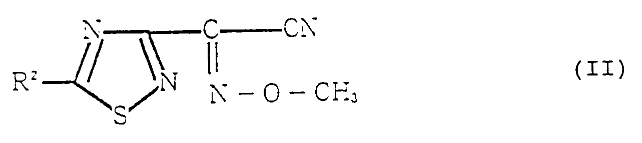 worin R² für eine geschützte Aminogruppe steht, oder ihrer Salze durch Einführen einer Aminoschutzgruppe in die Aminogruppe in die Position 5 einer Verbindung der Formel 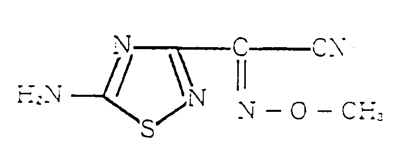 oder ihrer Salz und
die anschließende Hydrolyse einer Verbindung (II) oder ihrer Salze und erforderlichenfalls die Entfernung der Aminoschutzgruppe.

## Revendications

1. Procédé de préparation d'un composé répondant à la formule : 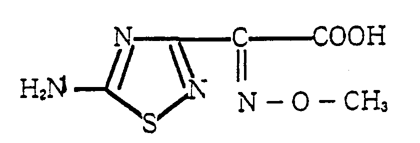 ou de ses sels, qui comprend le fait de former un composé répondant à la formule générale (II) : 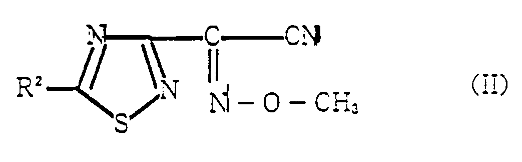 (dans laquelle R² représente un groupe amino protégé)
ou ses sels en introduisant un groupe protecteur du groupe amino dans le groupe amino à la position cinq d'un composé répondant à la formule : 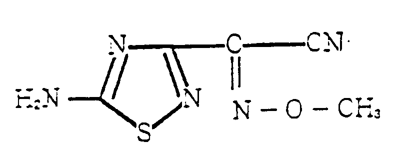 ou de ses sels, puis en hydrolysant un composé (II) ou ses sels, et si nécessaire, en éliminant le groupe protecteur du groupe amino.
